# FASCICULE DE BREVET EUROPEEN

(11) **EP 1 362 851 B1**
(45) Date de publication et mention de la délivrance du brevet: **24.08.2011**
(21) Numéro de dépôt: 03291041.6
(22) Date de dépôt: 29.04.2003
(51) Int. Cl.: A61K 8/40, A61Q 5/00

(54) **Composés diazirine-actifs photo-activables, compositions les contenant et leurs utilisations**
Photoaktivierbare Diazirinaktive Verbindungen, deren Zusammensetzungen und Verwendungen
Photo-activable diazirine-active compounds, compositions and uses thereof

(30) Priorité: 13.05.2002 FR 0205863
(43) Date de publication de la demande: 19.11.2003
(73) Titulaire: L'Oréal, 75008 Paris (FR)
(72) Inventeur: Vic, Gabin, 60280 Venette (FR); Livoreil, Aude, 75006 Paris (FR)
(74) Mandataire: Dodin, Catherine

(56) Documents cités:
- DE-C- 4 436 173
- US-A- 3 509 131
- US-A- 3 525 736
- US-A1- 2002 022 013
- HASHIMOTO, MAKOTO ET AL: "Versatile synthesis of phenoxydiazirine-based fatty acid analogs and photoreactive galactosylceramide" BIOORGANIC & MEDICINAL CHEMISTRY LETTERS (2001), VOLUME DATE 2002, 12(1), 89-91, 2001, XP002229065

## Description

La présente invention concerne des compositions cosmétiques contenant au moins un composé photo-activable, et les utilisations de ces composés et compositions photo-activables pour fixer des actifs cosmétiques sur les matières kératiniques.

Les produits mis en oeuvre pour fixer des agents cosmétiques sur les matières kératiniques présentent généralement l'inconvénient d'être éliminés très vite au lavage ou lors d'autres traitements.

Des procédés de coloration utilisant en tant qu'agent colorant des composés photo-activables sont connus de l'art antérieur. Par exemple la demande de brevet FR 2 605 220 décrit un procédé de coloration des matières kératiniques qui consiste à mettre les matières kératiniques en contact avec un azoture aromatique ou un azidoindole puis à exposer lesdites matières kératiniques à une source de lumière adéquate pour développer la couleur; la demande de brevet W01 06829 décrit le greffage d'une fonction photosensible de type phénylazoture sur un actif cosmétique.

On connaît également des procédés dans lesquels des polymères ou prépolymères sont déposés sur des fibres capillaires en présence de photoamorceurs puis ces fibres sont irradiées. Selon ce modèle, le brevet US 5 300 285 concerne plus particulièrement des silicones comprenant des groupes vinyliques et la demande de brevet WO 00 45777 des prépolymères constitués par un polyacrylate et/ou polyméthacrylate de polyol polyalkylenoxylé.

Le document "Hashimoto et al. Bioorganic and Medicinal Chemistry Letters (2001), Volume date 2002, 12(1), 89-91", décrit la synthèse d'analogues d'acides gras photoréactifs à groupement diazirine.

Le brevet DE4436173 décrit des surfaces matricielles hydrophobisées par des chaînes carbonées en C4-C12. Le brevet US3509131 décrit des amides carboxyliques substituées par des diazirines.

De manière générale, les procédés de l'art antérieur présentent les inconvénients suivants :
- le taux de greffage de l'actif cosmétique sur les matières kératiniques après irradiation reste faible : souvent le pourcentage en mole d'actifs cosmétiques greffés par rapport à la quantité d'actifs cosmétiques présents dans la solution utilisée est inférieur à 10%,
- de façon particulière, certains produits formés après irradiation tels que les nitrènes se réarrangent pour donner des composés de type ketenimines qui ne réagissent qu'avec des nucléophiles de type amine. Par conséquent, le taux de greffage de ces composés sur les matières kératiniques sera limité par la quantité de nucléophiles de type amine présents sur la surface à greffer,
- de plus, l'irradiation est généralement effectuée en utilisant des longueurs d'ondes voisines de 254 nm, cette irradiation de forte énergie a pour conséquence de dégrader les systèmes biologiques,
- de manière particulière, les groupes azotures aromatiques présentent une faible stabilité chimique, notamment en milieu réducteur ou oxydant. Par conséquent, un traitement mettant en oeuvre ce type de composés est difficilement compatible avec une permanente ou un défrisage.

Les inventeurs de la présente demande ont cherché une composition permettant de fixer de manière durable sur les matières kératiniques des actifs cosmétiques.

Le but de la présente demande est de proposer des composés qui permettent, de préférence en une seule étape, de fixer de manière durable un actif cosmétique sur les matières kératiniques.

De manière avantageuse et inattendue, ce but a été atteint par les inventeurs de la présente demande en utilisant des composés de type diazirine. Ces composés assurent la liaison entre l'agent cosmétique et les matières kératiniques : ils sont fixés à l'agent cosmétique et aux matières kératiniques par greffage covalent.

Les diazirines sont connues dans l'art antérieur en tant que marqueurs photochimiques.

Dans le cadre de la présente demande, les groupes fonctionnels de type diazirine présentent l'avantage de pouvoir être utilisés pour greffer un actif cosmétique sur les matières kératiniques sans nécessiter la présence de photo-amorceurs.

Ces diazirines permettent l'obtention d'un taux de greffage plus élevé que celui obtenu par les procédés selon l'art antérieur, ce procédé permet l'utilisation de longueurs d'ondes supérieures à 350 nm non dégradante pour les protéines.

Il a également été mis en évidence que les groupes diazirines présentent une très bonne stabilité chimique, même en milieu réducteur ou oxydant. En outre, les produits tels que les carbènes formés après irradiation entre 300 et 450 nm et de préférence entre 350 et 380 nm présentent l'avantage de s'insérer rapidement dans de nombreux types de liaisons (C-H, C-C, C=C, N-H, O-H, S-H) notamment présentes dans les matières kératiniques ou dans des éléments chimiques présents à la surface des matières kératiniques.

La présente invention a pour objet un composé photo-activable comprenant une diazirine liée de manière covalente à un actif cosmétique, ce composé est appelé "composé diazirine-actif".

Le composé diazirine-actif comprend un seul groupe photoactivable du type diazirine greffé par actif.

Ce composé permet d'assurer le greffage covalent d'actifs cosmétiques sur les matières kératiniques. De manière avantageuse, ce greffage s'effectue en une seule étape.

De manière préférée, la diazirine utilisée a pour formule I : 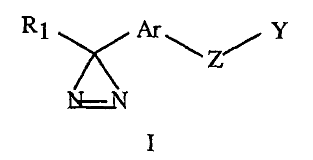 dans laquelle R₁ est choisi dans le groupe formé par un atome d'hydrogène, un radical alkyle en C₁ à C₁₀ linéaire ou ramifié, un radical alkényle en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', C≡N, COOH, F, Cl, Br, I, OR, COOR', SO₃H, COR', SH, SR', OH, où R' est un radical alkyle en C₁ à C₁₀,

Z est une simple liaison ou un groupe espaceur qui est une chaîne carbonée, linéaire ramifiée ou cyclique, saturée ou insaturée, en C₁-C₁₀₀, de préférence C₁-C₅₀, cette chaîne pouvant être interrompue par des hétéroatomes tels que le soufre, l'oxygène, l'azote, le silicium ou le phosphore et pouvant aussi comprendre un ou plusieurs substituants tels que des groupes hydroxyles, amines, thiols, carbamates, éthers, acides, esters, amides, cyano, uréido.

De manière préférée, il s'agira d'un polyol ou d'un polyalkylèneglycol (polyéthylèneglycol PEG ou polypropylèneglycol PPG).

Y est la fonction qui permet d'établir la liaison covalente entre la diazirine et l'actif cosmétique.

Y représente une fonction choisie dans le groupe formé par les alcools, amines, thiols, thiosulfates, acides carboxyliques et ses dérivés tels que les anhydrides, chlorures d'acide, esters, les acétals et hémi-acétals, les aminals et hémi-aminals, les cétones, les aldéhydes, alpha-hydroxycétones, les alpha-halocétones époxydes, les lactones, thiolactones, azalactones, isocyanate, thiocyanate, imines, imides (succinimides, glutimides), imidoesters, aziridines, imidates, oxazine et oxazoline, oxazinium et oxazolinium, les halogénes (fluor, chlore, iode, brome), les chlorotriazines, chloropyrimidines, chloroquinoxalines, chlorobenzotriazoles, les halogénures (X= F, Cl, I ou Br) de sulfonyle : SO₂X, les siloxanes, silanols, silanes, les pyridyldithio, les N-hydroxysuccinimide esters, les vinyles activés ou non activés parmi lesquels les acrylonitriles, esters acryliques et méthacryliques, acides et esters crotoniques, acides et esters cinnamiques, styrènes, butadiènes, éthers de vinyle, vinyle cétones, esters maléiques, maléimides, vinyl sulfones, les hydrazines, les phenyls glyoxals,

Ar représente un noyau aromatique choisi dans le groupe formé par : 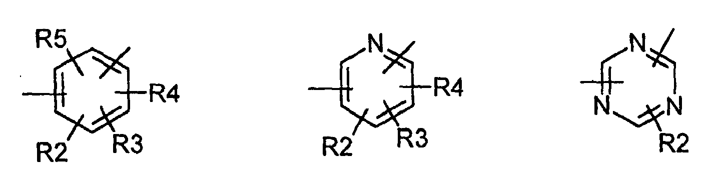 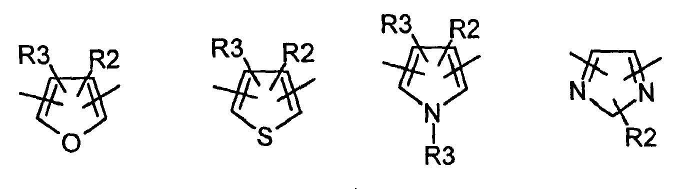 dans lesquels R₂, R₃, R₄, et R₅ représentent, indépendamment l'un de l'autre, des radicaux choisis dans le groupe formé par un atome d'hydrogène, un radical alkyle en C₁ à C₁₀ linéaire ou ramifié, un radical alkényle en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', C≡N, COOH, F, Cl, Br, I, OR', COOR', COR', SH, SR', OH, SO₃H où R' est un radical alkyle en C₁ à C_{10.}

Le composé diazirine-actif est synthétisé en formant une liaison covalente entre un groupe de la diazirine, de préférence le groupe Y de la diazirine de formule (I), et une fonction de l'actif cosmétique susceptible de former une liaison covalente avec ledit groupe de la diazirine. Cette liaison est établie par la mise en oeuvre de réactions chimiques classiques. Au besoin, la formation de la liaison peut être précédée d'un réaction visant à protéger un autre site de l'actif cosmétique que l'on ne souhaite pas voir réagir. Les réactions chimiques classiques de protection et de déprotection des groupes réactifs sont alors mises en oeuvre.

La présente demande a encore pour objet une composition cosmétique contenant, dans un solvant cosmétiquement acceptable, au moins un composé diazirine-actif. De manière préférée, le composé diazirine-actif comprend une diazirine de formule I.

Les compositions cosmétiques selon la présente invention comprennent au moins un solvant cosmétiquement acceptable tel que l'eau, l'éthanol. Ce solvant pourra également être un autre solvant organique tel que les alcanes en C₅ à C₁₀, l'acétone, la méthyléthylcétone, l'acétate de méthyle, l'acétate d'éthyle, l'acétate de butyle, le diméthoxyéthane, le diéthoxyéthane et leurs mélange.

Tous les actifs cosmétiques qui possèdent des groupements susceptibles de donner des réactions covalentes avec le groupe Y de la diazirine pourront être utilisés. Il s'agit notamment de polymères solubles naturels, les polymères solubles synthétiques, les polymères insolubles naturels, les polymères insolubles synthétiques, de particules minérales (métalliques ou non) ou organiques (latex, polystyrènes, silicones), de pigments, de filtres solaires, d'anti-oxydants, de colorants.

Par "polymères solubles ou insolubles" au sens de la présente demande, on entend solubles ou insolubles dans l'eau.

En tant qu'actif cosmétique sous forme de polymère on peut considérer par exemple les silicones, les polymères cationiques, les polymères amphotères.

En tant qu'actif cosmétique sous forme de particules minérales, on peut considérer les nacres, les pigments, ou bien encore les nanopigments (taille moyenne des particules primaires: généralement entre 5 nm et 100 nm, de préférence entre 10 nm et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium. On compte également l'alumine et/ou le stéarate d'aluminium.

En tant qu'actif cosmétique sous forme de filtres solaires, on peut considérer les dérivés 1,3,5-triazine, dérivés du dibenzoylméthane, les dérivés cinnamiques, les anthranilates ; les dérivés salicyliques, les dérivés du camphre ; les dérivés de la benzophénone ; les dérivés de β,β-diphénylacrylate, les dérivés de benzotriazole ; les dérivés de benzalmalonate ; les dérivés de benzimidazole ; les imadazolines ; les dérivés bis-benzoazolyle tels que décrits dans les brevets EP 669323 et US2,463,264 ; les dérivés de l'acide p-aminobenzoïque (PABA) ; les dérivés de méthylène bis-hydroxyphényl benzotriazole tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649, les 4,4-diarylbutadiènes tels que ceux décrits dans les demandes de brevet EP0967200 et DE19755649.

En tant qu'actif cosmétique sous forme de colorants, on peut considérer les composés choisis dans le groupe formé par les colorants benzèniques nitrés, les colorants aromatiques, les colorants amino-benzèniques, les colorants azoïques, les colorants anthraquinoniques, les diamines aromatiques, les aminophénols, les phénols et naphtols, les porphyrines, tétraphénylporphyrines, métalloporphyrines, les phtalocyanines, les caroténoides, les flavonoides, les molécules fluorescentes (fluoresceine, rhodamine, coumarine).

Les compositions cosmétiques selon la présente invention comprennent au moins une diazirine et au moins un actif cosmétique qui sont liés de manière covalente pour former un composé diazirine-actif.

La composition cosmétique comprend généralement de 0,001 à 90%, de préférence de 0,01 à 50% et de manière encore plus préférée de 0,1 à 10% en poids de composé diazirine-actif par rapport au poids total de la composition.

Les compositions selon l'invention peuvent également contenir d'autres constituants, agents cosmétiques qui ne sont pas liés à une molécule de diazirine.

En tant qu'agents cosmétiques de ce type, on peut utiliser les polymères, les particules minérales ou organiques, les filtres solaires les corps gras, les adoucissants, les antioxydants, les agents anti-radicaux libres, les émollients, les α-hydroxyacides, les agents hydratants, les vitamines, les agents répulsifs contre les insectes, les parfums, les anti-inflammatoires, les antagonistes de substance P, les charges, les colorants.

Lorsque les compositions selon l'invention comprennent au moins un autre agent cosmétique, celui-ci est présent en une quantité comprise entre 0,01 et 70%, de préférence de 0,1% à 50% en poids par rapport au poids total de la composition.

Ces compositions peuvent également contenir des activateurs des composés photo-activables comme les polyamines.

Ladite composition cosmétique peut être utilisée en tant qu'agent de coloration de la peau, des ongles ou des cheveux, en tant qu'agent hydratant, en tant qu'agent destiné à augmenter la brillance notamment des cheveux (agent brillant), en tant que filtre solaire, en tant qu'agent de conditionnement, en tant qu'agent de mise en forme des fibres kératiniques.

Les compositions cosmétiques selon l'invention peuvent être introduites dans toutes les formulations existant de traitement capillaire, par exemple dans des shampooings.

Par "matières kératiniques", au sens de la présente demande on entend les cheveux, cils, sourcils, poils, ongles, peau.

Un troisième objet de la présente demande consiste en l'utilisation d'un composé diazirine-actif, en particulier dans un procédé de traitement cosmétique. Il s'agit d'un procédé de traitement cosmétique des matières kératiniques, de préférence des matières kératiniques humaines telles que les cheveux, pour modifier les propriétés de ces matières kératiniques.

Un quatrième objet de la présente demande consiste en un procédé de traitement cosmétique dans lequel on applique sur les matières kératiniques un composé diazirine-actif selon l'invention et on expose lesdites matières kératiniques à un rayonnement d'une ou plusieurs longueurs d'ondes comprises entre 300 et 450 nm, de préférence 350 et 400 nm, et de manière encore plus préférée entre 350 et 380 nm.

Un cinquième objet de la présente demande consiste en l'utilisation de la composition cosmétique selon l'invention dans un procédé de traitement cosmétique et plus particulièrement en tant qu'agent de coloration, en tant qu'agent hydratant, en tant qu'agent destiné à augmenter la brillance notamment des cheveux (agent brillant), en tant que filtre solaire, en tant qu'agent de conditionnement, en tant qu'agent de mise en forme des fibres kératiniques.

Un sixième objet de la présente demande consiste en un procédé de traitement cosmétique dans lequel on applique sur les matières kératiniques une composition cosmétique selon l'invention et on expose lesdites matières kératiniques à un rayonnement d'une ou plusieurs longueurs d'ondes comprises entre 300 et 450 nm, de préférence 350 et 400 nm, et de manière encore plus préférée entre 350 et 380 nm. Ce procédé peut être réalisé en une ou plusieurs étapes.

Un septième objet de l'invention réside dans l'utilisation d'au moins une diazirine, en particulier une diazirine de formule I, dans un traitement cosmétique. De préférence, ce traitement cosmétique vise à modifier la réactivité chimique des matières kératiniques, de préférence les cheveux.

Un huitième objet de l'invention vise un procédé qui consiste à appliquer sur les matières kératiniques une composition contenant une diazirine de formule I, en présence éventuellement d'un actif cosmétique, et à exposer lesdites matières kératiniques à un rayonnement d'une ou plusieurs longueurs d'ondes comprises entre 300 et 450 nm, de préférence 350 et 380 nm.

Ce procédé qui met en oeuvre une diazirine pour greffer des groupes simples sur une surface permet de modifier la réactivité chimique de cette surface et donc de la préparer en vue d'un traitement ultérieur. Dans le cas des cheveux, cette technique peut être utilisée pour modifier spécifiquement certaines zones de la chevelure, par exemple, si l'on combine cette technique avec un traitement de coloration, on peut colorer spécifiquement certaines mèches d'une chevelure.

Une variante du huitième objet de l'invention consiste à appliquer sur les matières kératiniques de manière différée et dans un ordre indifférent une composition cosmétique contenant une diazirine de formule I et une composition contenant au moins un actif cosmétique, et à exposer lesdites matières kératiniques à un rayonnement d'une ou plusieurs longueurs d'ondes comprises entre 300 et 450 nm, de préférence 350 et 380 nm.

De manière préférentielle, après mise en contact de la ou des compositions avec les matières kératiniques, l'excès de composition est éliminé.

Les procédés selon la présente invention peuvent également être mis en oeuvre après un traitement préliminaire des matières kératiniques, ce traitement étant choisi dans le groupe formé par les traitements au moyen d'une composition réductrice, les permanentes, les colorations au moyen de colorants d'oxydation, les décolorations, les shampooings, les traitements coiffants.

Les exemples qui suivent illustrent l'invention sans en limiter la portée.

### EXEMPLES

### Exemple 1 : Synthèse d'un composé diazirine-actif

L'actif utilisé est un poly(oxyde d'éthylène / oxyde de propylène).

La synthèse est réalisée à partir d'un composé diazirine de formule (I) pour lequel R₁ est un groupement trifluorométhyle, Ar est un groupe phényle, Z est une liaison et Y est l'ester N-hydroxysuccinimidique d'un groupement acide carboxylique.

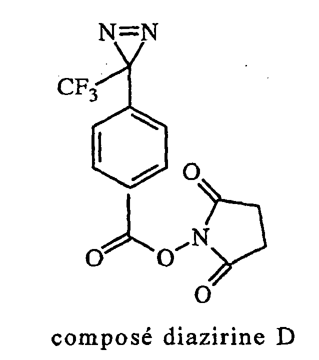

L'actif utilisé est un copolymère d'oxyde d'éthylène et d'oxyde de propylène portant une fonction amine terminale, la Jeffamine M-1000 commercialisée par TEXACO.

Pour réaliser cette synthèse, on plase sous agitation d'une solution aqueuse à 20% en masse de Jeffamine M-1000.

Le pH est ajusté à 8,5 avec une quantité nécessaire d'acide chlorhydrique ou de soude.

On réalise une solution de 2g de composé D dans 10ml de DMF. Cette solution est ajoutée lentement dans la solution de Jeffamine et laissée sous agitation, à température ambiante, pendant 7 heures.

On effectue ensuite une dialyse de la solution pendant 12h dans de l'eau distillée.

On obtient ainsi une solution aqueuse de composé diazirine-actif PEO/PPO/Diazirine, de teneur entre 15 et 20%, prête à l'emploi

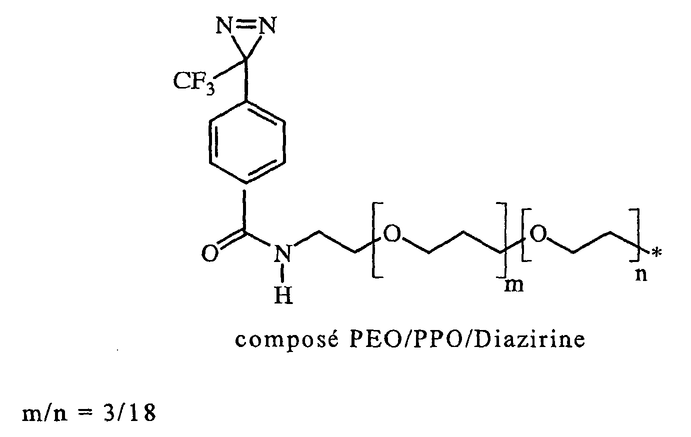

### Exemple 2 : Traitement capillaire avec un composé diazirine-actif

La solution de composé diazirine-actif PEO/PPO/Diazirine est appliquée sur mèches de cheveux naturels, à raison de 0,5ml de solution par mèches.

Les mèches sont séchées au sèche-cheveux jusqu'à évaporation de l'eau.

Les mèches sont ensuite irradiées à 360nm pendant 30 minutes.

### Exemple 3 : Traitement capillaire avec un composé diazirine

Une solution à 1% de composé diazirine D dans l'isododécane est appliquée sur cheveux.

On laisse l'isododécane s'évaporer, éventuellement en séchant les mèches au sèche-cheveux, puis on irradie les mèches à 360nm pendant 30 minutes.

## Revendications

1. Composition cosmétique contenant, dans un solvant cosmétiquement acceptable, au moins un composé diazirine-actif photoactivable comprenant une diazirine de formule (I) : 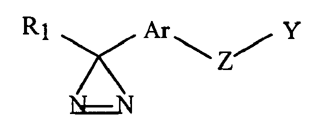 dans laquelle :
- R₁ est choisi dans le groupe formé par un atome d'hydrogène, un radical alkyle en C₁ à C₁₀ linéaire ou ramifié, un radical alkényle en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', C≡N, COOH, F, Cl, Br, I, OR, COOR', SO₃H, COR', SH, SR', OH, où R' est un radical alkyle en C₁ à C₁₀,
- Z est une simple liaison ou un groupe espaceur qui est une chaîne carbonée, linéaire, ramifiée ou cyclique, saturée ou insaturée, en C₁-C₁₀₀, cette chaîne pouvant être interrompue par des hétéroatomes et pouvant aussi comprendre un ou plusieurs substituants;
- Y est la fonction qui permet d'établir la liaison covalente entre la diazirine et l'actif cosmétique, et représente une fonction choisie dans le groupe formé par les alcools, amines, thiols, thiosulfates, acides carboxyliques et ses dérivés, esters, les acétals et hémi-acétals, les aminals et hémi-aminals, les cétones, les aldéhydes, alpha-hydroxycétones, les alpha-halocétones époxydes, les lactones, thiolactones, azalactones, isocyanate, thiocyanate, imines, imides, imidoesters, aziridines, imidates, oxazine et oxazoline, oxazinium et oxazolinium, les halogénes, les chlorotriazines, chloropyrimidines, chloroquinoxalines, chlorobenzotriazoles, les halogénures de sulfonyle SO₂X (X=F, Cl, I ou Br); les siloxanes, silanols, silanes, les pyridyldithio, les N-hydroxysuccinimide esters, les vinyles activés ou non activés;
les esters acryliques et méthacryliques, acides et esters crotoniques, acides et esters cinnamiques, styrènes, butadiènes, éthers de vinyle, vinyle cétones, esters maléiques, maléimides, vinyl sulfones, les hydrazines, les phenyls glyoxals,
- Ar représente un noyau aromatique choisi dans le groupe formé par : 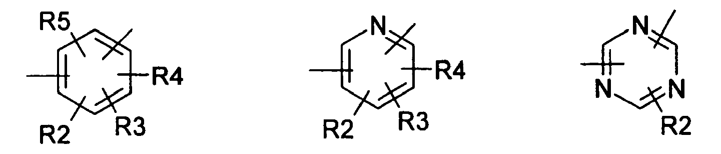 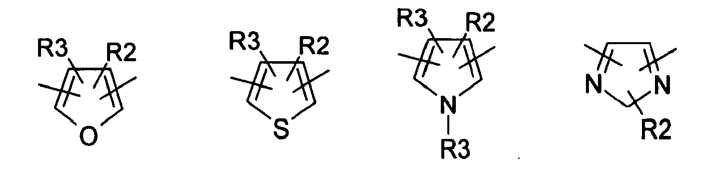 dans lesquels R₂, R₃, R₄, et R₅ représentent, indépendamment l'un de l'autre, des radicaux choisis dans le groupe formé par un atome d'hydrogène, un radical alkyle en C₁ à C₁₀ linéaire ou ramifié, un radical alkényle en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', C≡N, COOH, F, Cl, Br, I, OR', COOR', COR', SH, SR', OH, SO₃H où R' est un radical alkyle en C₁ à C₁₀;
ladite diazirine étant liée de manière covalente à un actif cosmétique choisi dans le groupe formé par les polymères solubles naturels, les polymères solubles synthétiques, les polymères insolubles naturels, les polymères insolubles synthétiques, les filtres solaires, les particules minérales, les antioxydants, les colorants.

2. Composition selon la revendication 1, dans laquelle l'actif cosmétique est un colorant choisi dans le groupe formé par les colorants benzéniques nitrés, les colorants aromatiques, les colorants amino-benzéniques, les colorants azoïques, les colorants anthraquinoniques, les diamines aromatiques, les aminophénols, les phénols et naphtols, les porphyrines, tétraphénylporphyrines, métalloporphyrines, les phtalocyanines, les caroténoides, les flavonoides, les molécules fluorescentes.

3. Composition selon l'une des revendications précédentes, comprenant de 0,001 à 90%, de préférence de 0,01 à 50% et de manière encore plus préférée 0,1 à 10% en poids de composé diazirine-actif par rapport au poids total de la composition.

4. Composition selon l'une des revendications précédentes, comprenant en outre au moins un agent choisi dans le groupe formé par les polymères, les particules minérales ou organiques, les filtres solaires, les colorants, les corps gras, les adoucissants, les antioxydants, les agents anti-radicaux libres, les émollients, les a-hydroxyacides, les agents hydratants, les vitamines, les agents répulsifs contre les insectes, les parfums, les anti-inflammatoires, les antagonistes de substance P, les charges.

5. Utilisation d'une diazirine dans un procédé de traitement cosmétique.

6. Utilisation selon la revendication 5, dans laquelle la diazirine est une diazirine de formule (I) telle que définie à la revendication 1.

7. Utilisation selon l'une des revendications 5 à 6, dans un procédé de traitement cosmétique des matières kératiniques, de préférence les cheveux, pour modifier la réactivité chimique de ces matières kératiniques.

8. Utilisation d'un composé diazirine-actif photoactivable tel que défini à l'une des revendications 1 à 2, dans un procédé de traitement cosmétique.

9. Procédé de traitement cosmétique **caractérisé en ce qu'**il consiste à appliquer sur les matières kératiniques un composé diazirine-actif photoactivable tel que défini à l'une des revendications 1 à 2, et à exposer lesdites matières kératiniques à un rayonnement d'une ou plusieurs longueurs d'ondes comprises entre 300 et 450 nm, de préférence 350 et 380 nm.

10. Utilisation de la composition cosmétique selon l'une des revendications 1 à 4, dans un procédé de traitement cosmétique.

11. Utilisation selon la revendication 10 en tant qu'agent de coloration, en tant qu'agent hydratant, en tant qu'agent destiné à augmenter la brillance notamment des cheveux (agent brillant), en tant que filtre solaire, en tant qu'agent de conditionnement, en tant qu'agent de mise en forme des fibres kératiniques.

12. Procédé de traitement cosmétique **caractérisé en ce qu'**il consiste à appliquer sur les matières kératiniques une composition cosmétique selon l'une des revendications 1 à 4, et à exposer lesdites matières kératiniques à un rayonnement d'une ou plusieurs longueurs d'ondes comprises entre 300 et 450 nm, de préférence 350 et 380 nm.

13. Procédé de traitement cosmétique **caractérisé en ce qu'**il consiste à appliquer sur les matières kératiniques une composition contenant une diazirine de formule (I) telle que définie à la revendication 1, et à exposer lesdites matières kératiniques à un rayonnement d'une ou plusieurs longueurs d'ondes comprises entre 300 et 450 nm, de préférence 350 et 380 nm.

14. Procédé de traitement cosmétique selon la revendication 13, **caractérisé en ce que** la composition contient au moins un actif cosmétique.

15. Procédé de traitement cosmétique selon la revendication 13, consistant à appliquer de manière différée et dans un ordre indifférent sur les matières kératiniques une composition cosmétique contenant une diazirine de formule (I) telle que définie à la revendication 1 et une composition contenant au moins un actif cosmétique puis à exposer lesdites matières kératiniques à un rayonnement d'une ou plusieurs longueurs d'ondes comprises entre 300 et 450 nm, de préférence 350 et 380 nm.

16. Procédé selon l'une des revendications 12 à 15 dans lequel, après mise en contact de la ou des compositions avec les matières kératiniques, l'excès de composition est éliminé.

17. Procédé selon l'une des revendications 12 à 16, comprenant un traitement préliminaire des matières kératiniques choisi dans le groupe formé par les traitements au moyen d'une composition réductrice, les permanentes, les colorations au moyen de colorants d'oxydation, les décolorations, les shampooings, les traitements coiffants.

## Claims

1. Cosmetic composition containing, in a cosmetically acceptable solvent, at least one photo-activatable diazirine-active agent compound comprising a diazirine of formula (I): 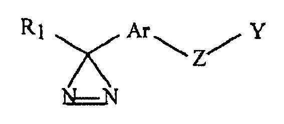 in which:
- R₁ is chosen from the group made up of a hydrogen atom, a linear or branched C₁ to C₁₀ alkyl radical, a linear or branched C₂ to C₁₀ alkenyl radical, a linear or branched C₂ to C₁₀ alkynyl, CF₃, CCl₃, CBr₃, NR'₃⁺, SR'₂⁺, SH₂⁺, NH₃⁺, NO₂, SO₂R', C=N, COOH, F, Cl, Br, I, OR', COOR', SO₃H, COR', SH, SR' and OH, where R' is a C₁ to C₁₀ alkyl radical,
- Z is a single bond or a spacer group which is a linear, branched or cyclic, saturated or unsaturated, C₁-C₁₀₀ carbon-based chain, it being possible for this chain to be interrupted with heteroatoms and to also comprise one or more substituents;
- Y is the function which makes it possible to establish the covalent bond between the diazirine and the cosmetic active agent, and represents a function chosen from the group made up of alcohols, amines, thiols, thiosulphates, carboxylic acids and derivatives thereof, esters, acetals and hemiacetals, aminals and hemiaminals, ketones, aldehydes, alpha-hydroxyketones, alpha-haloketone epoxides, lactones, thiolactones, azalactones, isocyanates, thiocyanates, imines, imides, imidoesters, aziridines, imidates, oxazines and oxazolines, oxaziniums and oxazoliniums, halogens, chlorotriazines, chloropyrimidines, chloroquinoxalines, chlorobenzotriazoles, sulphonyl halides SO₂ (X=F, Cl, I or Br) ; siloxanes, silanols, silanes, pyridyldithios, N-hydroxysuccinimide esters, activated or nonactivated vinyls; acrylic and methacrylic esters, crotonic acids and esters, cinnamic acids and esters, styrenes, butadienes, vinyl ethers, vinyl ketones, maleic esters, maleimides, vinyl sulphones, hydrazines and phenylglyoxals;
- Ar represents an aromatic nucleus chosen from the group made up of: 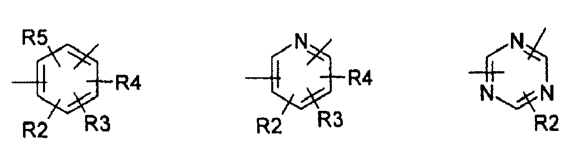 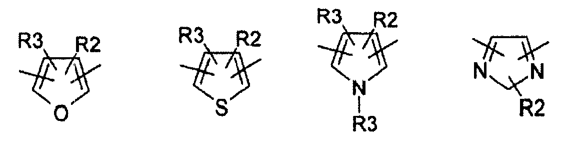 in which R₂, R₃, R₄ and R₅ represent, independently of one another, radicals chosen from the group made up of a hydrogen atom, a linear or branched C₁ to C₁₀ alkyl radical, a linear or branched C₂ to C₁₀ alkenyl radical, a linear or branched C₂ to C₁₀ alkynyl, CF₃, CCl₃, CBr₃, NR'₃⁺, SR'₂⁺, SH₂⁺, NH₃⁺, NO₂, SO₂R', C≡N, COOH, F, Cl, Br, I, OR', COOR', COR', SH, SR' OH and SO₃H, where R' is a C₁ to C₁₀ alkyl radical;
said diazirine being covalently bonded to a cosmetic active agent chosen from the group made up of natural soluble polymers, synthetic soluble polymers, natural insoluble polymers, synthetic insoluble polymers, sunscreens, mineral particles, antioxidants and dyes.

2. Composition according to Claim 1, in which the cosmetic active agent is a dye chosen from the group made up of nitrobenzene dyes, aromatic dyes, aminobenzene dyes, azo dyes, anthraquinone dyes, aromatic diamines, aminophenols, phenols and naphthols, porphyrins, tetraphenylporphyrins, metalloporphyrins, phthalocyanines, carotenoids, flavonoids and fluorescent molecules.

3. Composition according to either of the preceding claims, comprising from 0.001% to 90%, preferably from 0.01% to 50% and even more preferably from 0.1% to 10% by weight of diazirine-active agent compound, relative to the total weight of the composition.

4. Composition according to one of the preceding claims, also comprising at least one agent chosen from the group made up of polymers, mineral or organic particles, sunscreens, dyes, fatty substances, demulcents, antioxidants, free-radical scavengers, emollients, α-hydroxy acids, moisturizing agents, vitamins, insect repellents, fragrances, antiinflammatories, substance P antagonists, and fillers.

5. Use of a diazirine in a cosmetic treatment method.

6. Use according to Claim 5, in which the diazirine is a diazirine of formula (I) as defined in Claim 1.

7. Use according to either of Claims 5 and 6, in a cosmetic treatment method for keratin materials, preferably the hair, for modifying the chemical reactivity of these keratin materials.

8. Use of a photo-activatable diazirine-active agent compound as defined in either of Claims 1 and 2, in a cosmetic treatment method.

9. Cosmetic treatment method, **characterized in that** it consists in applying, to keratin materials, a photo-activatable diazirine-active agent compound as defined in either of Claims 1 and 2, and in exposing said keratin materials to radiation having one or more wavelengths between 300 and 450 nm, preferably 350 and 380 nm.

10. Use of the cosmetic composition according to one of Claims 1 to 4, in a cosmetic treatment method.

11. Use according to Claim 10, as a colouring agent, as a moisturizing agent, as an agent intended to increase the sheen in particular of the hair (sheen agent), as a sunscreen, as a conditioning agent, or as an agent for shaping keratin fibres.

12. Cosmetic treatment method, **characterized in that** it consists in applying, to keratin materials, a cosmetic composition according to one of Claims 1 to 4, and in exposing said keratin materials to radiation having one or more wavelengths of between 300 and 450 nm, preferably 350 and 380 nm.

13. Cosmetic treatment method, **characterized in that** it consists in applying, to keratin materials, a composition containing a diazirine of formula (I) as defined in Claim 1, and in exposing said keratin materials to radiation having one or more wavelengths of between 300 and 450 nm, preferably 350 and 380 nm.

14. Cosmetic treatment method according to Claim 13, **characterized in that** the composition contains at least one cosmetic active agent.

15. Cosmetic treatment method according to Claim 13, consisting in applying to keratin materials, with a delay and in any order, a cosmetic composition containing a diazirine of formula (I) as defined in Claim 1 and a composition containing at least one cosmetic active agent, and then in exposing said keratin materials to radiation having one or more wavelengths of between 300 and 450 nm, preferably 350 and 380 nm.

16. Method according to one of Claims 12 to 15, in which, after the composition(s) has (have) been brought into contact with the keratin materials, the excess composition is removed.

17. Method according to one of Claims 12 to 16, comprising a preliminary treatment of the keratin materials, chosen from the group made up of treatments by means of a reducing composition, permanent waves, dyeing by means of oxidation dyes, bleaching, shampooing and styling treatments.

## Patentansprüche

1. Kosmetikzusammensetzung, die in einem kosmetisch unbedenklichen Lösungsmittel mindestens eine fotoaktivierbare diazirinaktive Verbindung, umfassend ein Diazirin der Formel (I) 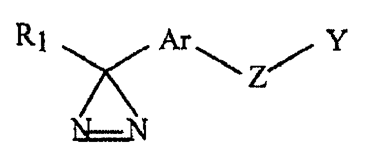 enthält, in der:
- R₁ aus der Gruppe Wasserstoffatom, geradkettiger oder verzweigter C₁- bis C₁₀-Alkylrest, geradkettiger oder verzweigter C₂- bis C₁₀-Alkenylrest, geradkettiges oder verzweigtes C₂- bis C₁₀-Alkinyl, CF₃, CCl₃, CBr₃, NR'₃+, SR'₂+, SH₂+, NH₃+, NO₂, SO₂R', C=N, COOH, F, Cl, Br, I, OR, COOR', SO₃H, COR', SH, SR', OH, wobei R' einen C₁- bis C₁₀-Alkylrest bedeutet, stammt,
- Z eine Einfachbindung oder eine Spacer-Gruppe bedeutet, die eine geradkettige, verzweigte oder cyclische, gesättigte oder ungesättigte C₁-C₁₀₀-Kohlenstoffkette ist, wobei diese Kette durch Heteroatome unterbrochen sein kann und auch einen oder mehrere Substituenten umfassen kann;
- Y die Funktion ist, die die Bildung der covalenten Bindung zwischen dem Diazirin und dem Kosmetikwirkstoff gestattet und eine Funktion aus der Gruppe Alkohole, Amine, Thiole, Thiosulfate, Carbonsäuren und ihre Derivate, Ester, Acetale und Hemiacetale, Aminale und Hemiaminale, Ketone, Aldehyde, alpha-Hydroxyketone, alpha-Halogenketone, Epoxide, Lactone, Thiolactone, Azalactone, Isocyanat, Thiocyanat, Imine, Imide, Imidoester, Aziridine, Imidate, Oxazin und Oxazolin, Oxazinium und Oxazolinium, Halogene, Chlortriazine, Chlorpyrimidine, Chlorchinoxaline, Chlorbenzotriazole, Sulfonylhalogenide SO₂X (X=F, Cl, I oder Br); Siloxane, Silanole, Silane, Pyridyldithio, N-Hydroxysuccinimidester, aktivierte oder nicht aktivierte Vinyle; Acrylsäure- und Methacrylsäureester, Crotonsäuren und -ester, Zimtsäuren und -ester, Styrole, Butadiene, Vinylether, Vinylketone, Maleinsäureester, Maleimide, Vinylsulfone, Hydrazine, Phenylglyoxale,
- Ar einen aromatischen Ring aus der Gruppe 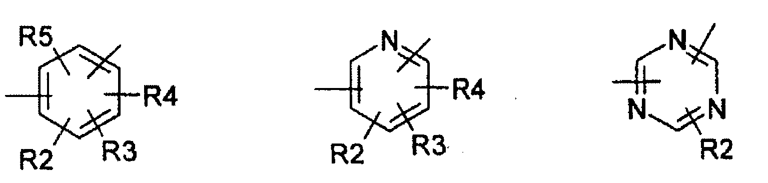 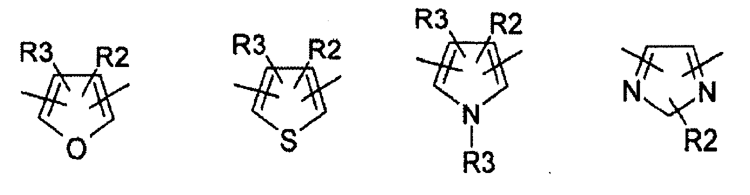 bedeutet, worin R₂, R₃, R₄ und R₅ unabhängig voneinander Reste aus der Gruppe Wasserstoffatom, geradkettiger oder verzweigter C₁- bis C₁₀-Alkylrest, geradkettiger oder verzweigter C₂- bis C₁₀-Alkenylrest, geradkettiges oder verzweigtes C₂- bis C₁₀-Alkinyl, CF₃, CCl₃, CBr₃, NR'₃+, SR'₂+, SH₂+, NH₃+, NO₂, SO₂R', C=N, COOH, F, Cl, Br, I, OR', COOR', COR', SH, SR', OH, SO₃H, wobei R' einen C₁- bis C₁₀-Alkylrest bedeutet, stammt,
wobei das Diazirin covalent an einen Kosmetikwirkstoff aus der Gruppe natürliche lösliche Polymere, synthetische lösliche Polymere, natürliche unlösliche Polymere, synthetische unlösliche Polymere, Sonnenschutzfilter, mineralische Partikel, Antioxidantien, Farbstoffe gebunden ist.

2. Zusammensetzung nach Anspruch 1, wobei es sich bei dem Kosmetikwirkstoff um einen Farbstoff aus der Gruppe Nitrobenzolfarbstoffe, aromatische Farbstoffe, Aminobenzolfarbstoffe, Azofarbstoffe, Anthrachinonfarbstoffe, aromatische Diamine, Aminophenole, Phenole und Naphthole, Porphyrine, Tetraphenylporphyrine, Metalloporphyrine, Phthalocyanine, Carotinoide, Flavonoide und fluoreszierende Moleküle handelt.

3. Zusammensetzung nach einem der vorhergehenden Ansprüche, umfassend 0,001 bis 90% Gew.-%, bevorzugt 0,01 bis 50 Gew.-% und noch stärker bevorzugt 0,1 bis 10 Gew.-% diazirinaktive Verbindung in Bezug auf das Gesamtgewicht der Zusammensetzung.

4. Zusammensetzung nach einem der vorhergehenden Ansprüche, die weiterhin mindestens ein Mittel aus der Gruppe Polymere, mineralische oder organische Partikel, Sonnenschutzfilter, Farbstoffe, Fettkörper, zartmachende Substanzen, Antioxidantien, Mittel gegen freie Radikale, Emollientien, α-Hydroxysäuren, feuchtigkeitsspendende Mittel, Vitamine, Insektenabwehrmittel, Duftstoffe, entzündungshemmende Mittel, Substanz-P-Antagonisten, Ladungen umfasst.

5. Verwendung eines Diazirins in einem Kosmetikbehandlungsverfahren.

6. Verwendung nach Anspruch 5, wobei es sich bei dem Diazirin um ein Diazirin der Formel (I) wie in Anspruch 1 definiert handelt.

7. Verwendung nach einem der Ansprüche 5 bis 6 in einem Kosmetikbehandlungsverfahren für Keratinsubstanzen, vorzugsweise das Haar, um die chemische Reaktivität dieser Keratinsubstanzen zu modifizieren.

8. Verwendung einer fotoaktivierbaren diazirinaktiven Verbindung wie in einem der Ansprüche 1 bis 2 definiert in einem Kosmetikbehandlungsverfahren.

9. Kosmetikbehandlungsverfahren, **dadurch gekennzeichnet, dass** es darin besteht, dass man eine diazirinaktive Verbindung wie in einem der Ansprüche 1 bis 2 definiert auf die Keratinsubstanzen aufbringt und die Keratinsubstanzen einer Strahlung von einer oder mehreren Wellenlängen zwischen 300 und 450 nm, vorzugsweise 350 und 380 nm, aussetzt.

10. Verwendung der Kosmetikzusammensetzung nach einem der Ansprüche 1 bis 4 in einem kosmetischen Behandlungsverfahren.

11. Verwendung nach Anspruch 10 als Färbemittel, als feuchtigkeitsspendendes Mittel, als Glanzverbesserungsmittel, insbesondere für das Haar (Glanzmittel), als Sonnenschutzfilter, als Conditioner, als Formungsmittel für Keratinfasern.

12. Kosmetikbehandlungsverfahren, **dadurch gekennzeichnet, dass** es darin besteht, dass man auf die Keratinsubstanzen eine Kosmetikzusammensetzung nach einem der Ansprüche 1 bis 4 aufbringt und die Keratinsubstanzen einer Strahlung von einer oder mehreren Wellenlängen zwischen 300 bis 450 nm, vorzugsweise 350 und 380 nm, aussetzt.

13. Kosmetikbehandlungsverfahren, **dadurch gekennzeichnet, dass** es darin besteht, dass man eine Zusammensetzung, enthaltend ein Diazirin der Formel (I) wie in Anspruch 1 definiert auf die Keratinsubstanzen aufbringt und die Keratinsubstanzen einer Strahlung von einer oder mehreren Wellenlängen zwischen 300 und 450 nm, vorzugsweise 350 und 380 nm, aussetzt.

14. Kosmetikbehandlungsverfahren nach Anspruch 13, **dadurch gekennzeichnet, dass** die Zusammensetzung mindestens einen Kosmetikwirkstoff enthält.

15. Kosmetikbehandlungsverfahren nach Anspruch 13, **dadurch gekennzeichnet, dass** man differenziert und in beliebiger Reihenfolge auf die Keratinsubstanzen eine Kosmetikzusammensetzung, enthaltend ein Diazirin der Formel (I) wie in Anspruch 1 definiert und eine Zusammensetzung, die mindestens einen Kosmetikwirkstoff enthält, auf die Keratinsubstanzen aufbringt und dann diese Keratinsubstanzen einer Strahlung von einer oder mehreren Wellenlängen zwischen 300 und 450 nm, vorzugsweise 350 und 380 nm, aussetzt.

16. Verfahren nach einem der Ansprüche 12 bis 15 bei dem nach dem Inkontaktbringen der Zusammensetzung(en) mit den Keratinsubstanzen die überschüssige Zusammensetzung entfernt wird.

17. Verfahren nach einem der Ansprüche 12 bis 16, umfassend eine Vorbehandlung der Keratinsubstanzen aus der Gruppe bestehend aus den Behandlungen mit einer reduzierenden Zusammensetzung, Dauerwellen, Färben mit Oxidationsfarbstoffen, Entfärben, Waschen mit Shampoo, Frisierbehandlungen.
